# EUROPÄISCHE PATENTSCHRIFT

(11) **EP 0 954 239 B1**
(45) Veröffentlichungstag und Bekanntmachung des Hinweises auf die Patenterteilung: **21.04.2004**
(21) Anmeldenummer: 98905299.8
(22) Anmeldetag: 15.01.1998
(51) Int. Cl.: A61B 5/00

(54) **MESSANORDNUNG ZUR BESTIMMUNG DER SCHUBLADENVERSCHIEBUNG**
MEASURING DEVICE FOR DETERMINING DRAWER DISPLACEMENT
DISPOSITIF DE MESURE POUR LA DETERMINATION DU TIROIR

(30) Priorität: 21.01.1997 DE 19701838
(43) Veröffentlichungstag der Anmeldung: 10.11.1999
(73) Patentinhaber: Gollhofer, Albert Professor Dr., 79252 Stegen (DE)
(72) Erfinder: Gollhofer, Albert Professor Dr., 79252 Stegen (DE)
(74) Vertreter: Pfiz, Thomas, Dr.
(86) Internationale Anmeldenummer: PCT/EP1998/000207
(87) Internationale Veröffentlichungsnummer: WO 1998/031274

(56) Entgegenhaltungen:
- EP-A- 0 074 407
- EP-A- 0 710 466
- WO-A-87/05789
- DE-C- 19 617 981
- US-A- 4 583 554
- US-A- 4 804 000

## Beschreibung

Die Erfindung betrifft eine Meßanordnung zur Bestimmung der Schubladenverschiebung der Tibia gegenüber dem Femur an einem Bein eines Probanden.

Die Tibia läßt sich in der Verspannungsstruktur des Kniegelenks in sogenannten Schubladenbewegungen gegenüber dem Oberschenkel schubladenartig nach vorn oder hinten schieben, wobei die Femurkondylen eine Gleitbewegung auf dem zugewandten Tibiaplateau vollführen. Solche Gleit- oder Translationsbewegungen treten neben einer einfachen Abrollbewegung auch bei der aktiven Extension/Flexion des Kniegelenks auf und spiegeln dessen ligamentären Zustand wieder. Zur klinischen Untersuchung der Schubladenphänomene ist der sogenannte "Lachmann-Test" bekannt, bei dem ein Untersucher Ober- und Unterschenkel eines in Rückenlage liegenden Patienten kniegelenknah umfaßt und den Schienbeinkopf im Sinne einer ventralen Schublade unter Kraftausübung nach vorne zieht. Dieser Test ist subjektiv und liefert keine quantitativen Ergebnisse über das Ausmaß einer Schublade. Er eignet sich insbesondere nicht für systematische Untersuchungen des Zusammenhangs zwischen der Schubladenfreiheit und dem Zustand der Bandkomplexe.

Eine Meßanordnung zur Bestimmung der Schubladenverschiebung der Tibia gegenüber dem Femur ist z.B. aus WO-A-8 705 789 bekannt. In WO-A-8 705 789 wird auf die Anwendung zweier unterschiedlicher Sensoren hingerwiesen, wobei die Schubladenverschiebung sich aus den zwei absoluten Messungen berechnen läßt.

Ausgehend hiervon liegt der Erfindung die Aufgabe zugrunde, eine objektive Messung der Schubladenverschiebung am Kniegelenk insbesondere auch in funktionellen Situationen, wie beim Stehen, Gehen und Laufen, zu ermöglichen.

Diese Aufgabe wird nach der Erfindung durch die Merkmale des unabhängigen Patentanspruchs 1 gelöst. Vorteilhafte Ausgestaltungen und Weiterbildungen der Erfindung ergeben sich aus den abhängigen Ansprüchen.

Die Erfindung geht von dem Gedanken aus, sowohl die räumliche Lage des Femurs als auch der Tibia im Kniegelenksbereich durch gesonderte Messungen in einem gemeinsamen Bezugssystem zu erfassen. Um dies zu ermöglichen, sind gemäß der Erfindung ein am Unterschenkel eines Beines fixierbares Referenzgestell, zwei an dem Referenzgestell im Abstand voneinander gestellfest angeordnete, im Bereich der proximalen Tibia und des distalen Femurs gegen die Vorderseite des Beines ausrichtbare Abstandssensoren, und eine mit den Ausgangssignalen der Abstandssensoren beaufschlagbare Auswerteeinrichtung zur Bestimmung der Schubladenverschiebung vorgesehen. Damit lassen sich objektive, exakte Meßwerte sowohl bei von außen ausgelösten passiven als auch bei aktiven Schubladenbewegungen gewinnen, ohne daß es erforderlich wäre, das Bein zu immobilisieren. Auch muß das Referenzgestell aufgrund der parallelen Erfassung der Ausgangssignale beider Abstandssensoren nicht vollkommen starr in Verbindung mit dem Unterschenkel stehen, was insbesondere bei aktiven Schubladentests ohnehin nur unzureichend möglich ist.

Vorteilhafterweise ist ein Abstandssensor gegen die vordere Kniescheibenfläche (Facies anterior patellae) als äußere Bezugsfläche für die Lage der Femurrollen und der andere Abstandssensor gegen die vordere Schienbeinrauhigkeit (Tuberositas tibiae) als Bezugsfläche für die Lage des Tibiakopfes ausgerichtet.

Um die Ausgangssignale der Abstandssensoren in direkte Beziehung zu den zu messenden Transversalverschiebungen setzen zu können, weisen die Abstandssensoren zueinander parallele Meßstrecken auf, die parallel zum Tibiaplateau in im wesentlichen antero-posteriorer Richtung verlaufen. Damit läßt sich die Schubladenverschiebung nach Maßgabe eines Differenzwertes der Ausgangssignale der Abstandssensoren in einfacher Weise bestimmen.

Zur Einstellung einer geeigneten Meßposition sind die Abstandssensoren vorzugsweise in allen drei Raumrichtungen begrenzt verschiebbar und in ihrer Verschiebelage gestellfest arretierbar an dem Referenzgestell angeordnet.

Die Abstandssensoren können einen kapazitiven, induktiven oder als Ohmscher Widerstand ausgebildeten Wegmeßsensor aufweisen, dessen Meßstrecke gegenüber einem gestellfesten Bezugspunkt festgelegt ist. Damit läßt sich die jeweilige momentane räumliche Lage der sensierten Beinflächen in einem durch das Referenzgestell gebildeten gemeinsamen Bezugssystem erfassen. In einer einfachen Ausführung sind die Abstandssensoren als Linearpotentiometer ausgebildet, deren Schleifer mit ihren freien Enden federbelastet an die Vorderseite des Beines andrückbar sind.

Alternativ können die Abstandssensoren zur berührungslosen Messung als Ultraschall-, Infrarot- oder Lasersensoren ausgebildet sein.

Vorteilhafterweise ist das Referenzgestell über ein Stützpolster an der Vorderseite des Unterschenkels abstützbar. Das Stützpolster ermöglicht eine weitgehend schmerzfreie anatomische Anpassung, wobei insbesondere Schaumstoffe, wie Styropor oder Polyurethan günstige Eigenschaften aufweisen. Das Referenzgestell ist geeignet leicht gebaut, um am Unterschenkel getragen zu werden, wobei es mittels zirkulär um den Unterschenkel spannbarer Klettverschlußbänder fixierbar ist. Mit diesen Maßnahmen kann in vorteilhafter Weise eine starre Befestigung des Referenzgestells am Unterschenkel sichergestellt werden, so daß sich Relativbewegungen des Referenzgestells weitgehend vermeiden lassen.

Vorteilhafterweise lassen sich äußere Kräfte über eine vorzugsweise eine Bandschlinge aufweisende Kraftübertragungsvorrichtung in den Unterschenkel einleiten, wobei die aufgeprägte Kraft betragsmäßig durch einen Kraftaufnehmer bestimmbar ist.

Bei mobilen Ausführungen ist es vorteilhaft, wenn die Auswerteeinrichtung eine tragbare Anzeigeeinheit aufweist, mittels welcher die erfaßten Werte der Abstandssensoren auf einem LCD-Display darstellbar sind. Dabei kann es ausreichen, wenn lediglich die während eines Meßzyklus aufgenommenen Maximalwerte angezeigt werden. Zur drahtlosen Verbindung der Abstandssensoren mit der Auswerteeinrichtung kann eine Telemetrieeinrichtung vorgesehen sein.

Weiter ist es von Vorteil, wenn die Auswerteeinrichtung einen über einen Analog/Digital-Wandler mit den digitalisierten Ausgangssignalen der Abstandssensoren und gegebenenfalls den Ausgangssignalen eines Kraftaufnehmers beaufschlagbaren Mikrocomputer aufweist. Damit läßt sich die Datenerhebung in für wissenschaftliche Zwecke geeigneter Weise durchführen.

Zur zusätzlichen Untersuchung muskulärer Aktivierungen lassen sich elektromyographische Signale an Oberflächenelektroden ableiten, wobei insbesondere der M. biceps femoris im Oberschenkelbereich und der M. gastrocnemius im Unterschenkelbereich als Abgriffstellen geeignet sind.

Um eventuelle Rotationsbewegungen des Referenzgestells gegenüber dem Unterschenkel erfassen zu können, sind vorteilhafterweise mindestens drei Abstandssensoren an dem Referenzgestell angeordnet, wobei ein erster Abstandssensor im proximalen Tibiabereich und ein zweiter Abstandssensor im distalen Femurbereich gegen die Vorderseite des Beines ausgerichtet sind, und wobei mindestens ein weiterer Abstandssensor im horizontalen und/ oder vertikalen Abstand von dem zweiten Abstandssensor gegen den Unterschenkel ausgerichtet ist.

Im folgenden wird die Erfindung anhand des in der Zeichnung in schematischer Weise dargestellten Ausführungsbeispiels näher erläutert. Es zeigen
- Fig. 1 und 2: eine an einem Bein fixierte Meßanordnung zur Bestimmung der Schubladenverschiebung in lateraler und frontaler Ansicht;
- Fig. 3: ein Blockschaltbild von Elementen zur Datenerfassung und -verarbeitung der Meßanordnung; und
- Fig. 4: ein Zeitdiagramm verschiedener mit der Meßanordnung gewonnener Meßsignale.

Die in der Zeichnung dargestellte Meßanordnung besteht im wesentlichen aus einem Referenzgestell 10, zwei an dem Referenzgestell 10 im Abstand voneinander gestellfest angeordneten Abstandssensoren 12, 14 sowie einer mit den Ausgangssignalen der Abstandssensoren 12, 14 beaufschlagbaren Auswerteeinrichtung 16 zur Bestimmung der Schubladenverschiebung.

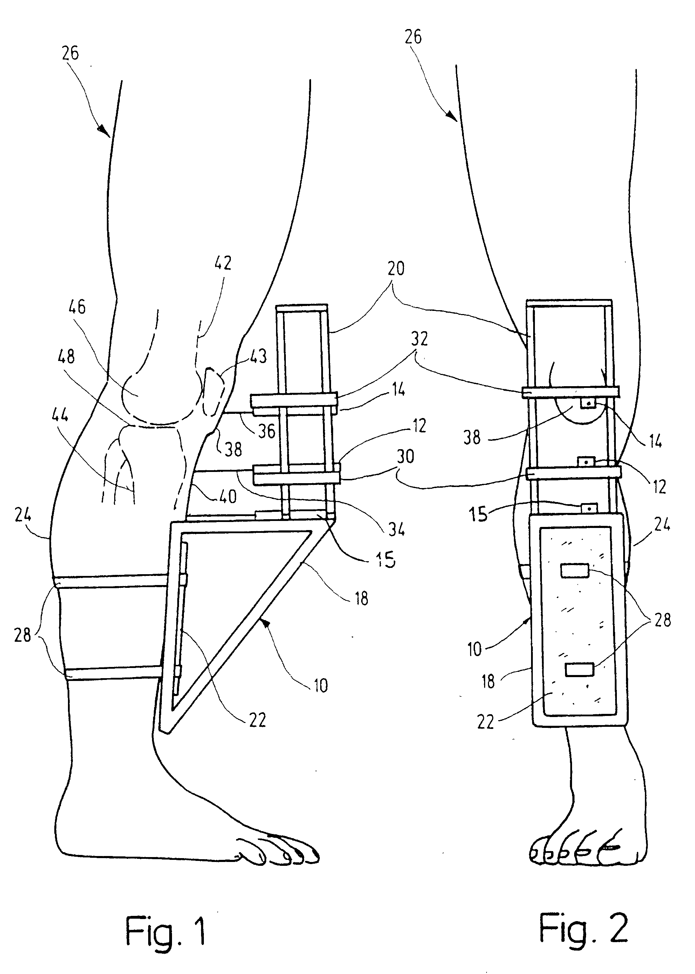

Wie in Fig. 1 und 2 gezeigt, weist das Referenzgestell 10 einen gerüstartigen Aufbau auf, wobei eine untere Tragkonstruktion 18 eine Plattform für einen Stativaufbau 20 bildet. Die Tragkonstruktion 18 ist über ein Stützpolster 22 aus Styropor an der Vorderseite des Unterschenkels 24 eines Beines 26 abstützbar und mittels Klettverschlußbändern 28 fixierbar. Um eine Verdrehung um die Unterschenkelachse zu verhindern, können zusätzliche Stützstreben vorgesehen sein, welche im Bereich des Schienbeinkopfs lateral und medial angreifen.

An dem Stativaufbau 20 sind zwei in einer Transversalebene ausgerichtete Montageplatten 30, 32 vertikal verschiebbar angeordnet, auf denen die Abstandssensoren 12, 14 horizontal verschiebbar und in einer eingestellten Verschiebelage arretierbar gelagert sind.

Die Abstandssensoren 12, 14 sind als Linearpotentiometer ausgebildet, deren Schleifer über Taststifte 34, 36 gegenüber einem gestellfesten Linearwiderstand verschiebbar sind, wobei die Taststifte 34, 36 in Richtung ihrer freien Enden federbelastet sind.

Zur Ausmessung von Schubladenbewegungen werden die Linearpotentiometer 12, 14 in ihrer Lage bezüglich des Kniegelenks so orientiert, daß der obere Taststift 36 gegen die vordere Kniescheibenfläche 38 und der untere Taststift gegen die vordere Schienbeinrauhigkeit 40 angedrückt wird. Diese äußeren Beinregionen dienen als Bezugsfläche für den distalen Abschnitt des Femurs 42 bzw. den proximalen Abschnitt der Tibia 44 (Fig. 1). Bei einer hinteren oder vorderen Schubladenverschiebung gleiten die Femurkondylen 46 auf dem Tibiaplateau 48 unter gegenseitiger Relativverschiebung in antero-posteriorer Richtung. Entsprechend sind die Taststifte 34, 36 ausgerichtet, so daß die Verschiebewege des Femurs 42 und der Tibia 44 direkt gegenüber dem Referenzgestell 10 meßbar sind.

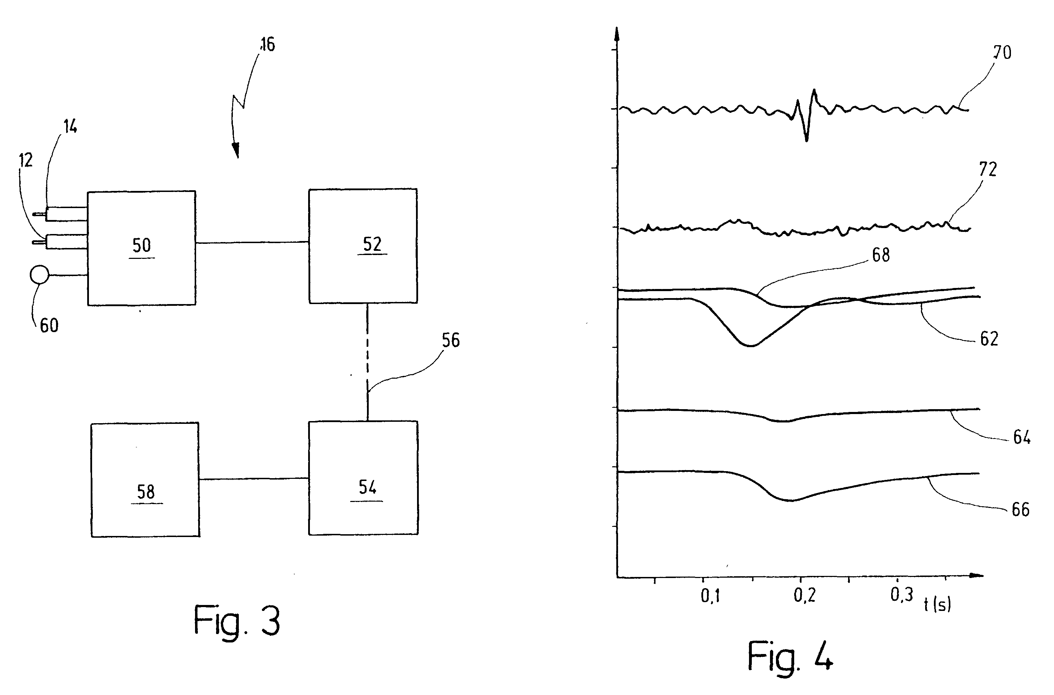

Grundsätzlich ist eine möglichst starre Fixierung des Referenzgestells 10 an dem Unterschenkel 24 von Vorteil. Um dennoch auftretende Rotationsbewegungen des Referenzgestells 10 gegenüber dem Unterschenkel 24 erfassen zu können, ist mindestens ein weiterer Abstandssensor 15 vorgesehen. Wenn dieser, wie in Fig. 3 gezeigt, entlang der Längsachse der Tibia 44 im vertikalen Abstand von dem Abstandssensor 12 angeordnet ist, läßt sich eine mögliche Rotations- bzw. Kippbewegung in der Sagittalebene erfassen. Wenn ein weiterer Abstandssensor (nicht gezeigt) in gleicher Höhe der Tibia 44 im horizontalen Abstand neben dem Abstandssensor 12 angeordnet ist, läßt sich auch eine Rotation des Referenzgestells 10 um die Achse der Tibia 44 erfassen.

Wie aus Fig. 3 ersichtlich, werden die Ausgangssignale der Potentiometer 12, 14 in einer Signalaufbereitungseinheit 50 verstärkt und ggf. digitalisiert sowie über ein LCD-Display angezeigt, wobei die Einheit 50 zweckmäßig am Körper des Probanden angeordnet ist. Die Daten lassen sich über ein Sendemodul 52 und ein Empfangsmodul 54 einer Telemetrieeinrichtung über eine Funkstrecke 56 zu einer stationären Verarbeitungseinheit 58 übertragen. In einer Laborsituation kann die Signalaufbereitungseinheit 50 direkt mit der durch einen Personalcomputer gebildeten Verarbeitungseinheit 58 verbunden sein, wobei dann die Digitalisierung der Daten in einem als Einsteckkarte ausgebildetem Analog-Digital-Wandler in dem Personalcomputer erfolgt.

Passive Schubladenbewegungen lassen sich unter der Einwirkung äußerer Kräfte beobachten. Um reproduzierbare Meßbedingungen zu schaffen, kann dabei ein Kraftaufnehmer 60 vorgesehen sein, mittels welchem die angewandten Kräfte meßbar und damit der weiteren Auswertung zugänglich sind. Weiter kann zur Erhöhung des Reproduzierbarkeitsgrades der Messungen ein nicht gezeigtes Winkelmeßsystem zur Erfassung der Beuge- bzw. Streckstellung des Kniegelenks vorgesehen sein.

Bei Schubladenbewegungen werden vermutlich von Mechanorezeptoren Muskelreflexe zum Schutz des Kniegelenks vor Extremstellungen ausgelöst. Neben der Messung der rein mechanischen Verschiebungen ist es daher auch von Interesse, Signale des neuromuskulären Systems, wie sie in Form von Elektromyogrammen mittels Oberflächenelektroden ableitbar sind, aufzuzeichnen.

In Fig. 4 sind die zeitaufgelöst aufgezeichneten Signalamplituden der verschiedenen Meßsensoren in willkürlichen Einheiten über der Zeit dargestellt. Die Kurve 62 gibt den mit dem Kraftaufnehmer 60 erfaßten, in den Unterschenkel 24 eingeleiteten Kraftimpuls wieder. Anhand des Signals 64 des Linearpotentiometers 12 ist eine geringe zeitlich verzögerte Bewegung der Tibia 44 gegenüber dem Referenzgestell 10 zu erkennen, während das Signal 66 des Potentiometers 14 die Bewegung der Patella 43 und damit mittelbar des Femurs 46 zeigt. Die eigentliche Schubladenverschiebung ergibt sich schließlich aus der Differenzkurve 68 der Signalverläufe 64 und 66. Daneben sind in Fig. 4 auch die zeitgleich abgegriffenen EMG-Signale von biceps femoris (Kurve 70) und gastrocnemius (Kurve 72) dargestellt, welche in Zusammenhang mit der Krafteinleitung stehende Aktivierungen zeigen.

Zusammenfassend ist folgendes festzustellen: Die Erfindung betrifft eine Meßanordnung zur Bestimmung der Schubladenverschiebung der Tibia 44 gegenüber dem Femur 42 an einem Bein 26 eines Probanden, mit einem am Unterschenkel 24 des Beines 26 fixierbaren Referenzgestell 10, zwei an dem Referenzgestell 10 im Abstand voneinander gestellfest angeordneten, im proximalen Tibiabereich und distalen Femurbereich gegen die Vorderseite des Beines 26 ausrichtbaren Abstandssensoren 12,14 und einer mit den Ausgangssignalen der Abstandssensoren 12,14 beaufschlagbaren Auswerteeinrichtung 16. Die Schubladenverschiebung wird dabei im wesentlichen unabhängig von überlagerten Relativbewegungen zwischen Unterschenkel und Referenzgestell 10 als Differenzwert der beiden zeitgleich erfaßten Ausgangssignale der Abstandssensoren 12, 14 bestimmt.

## Patentansprüche

1. Meßanordnung zur Bestimmung der Schubladenverschiebung der Tibia (44) gegenüber dem Femur (42) an einem Bein (26) eines Probanden mit einem am Unterschenkel (24) des Beines (26) fixierbaren Referenzgestell (10), **gekennzeichnet durch**
- zwei an dem Referenzgestell (10) im Abstand voneinander gestellfest angeordnete, im proximalen Tibiabereich und distalen Femurbereich gegen die Vorderseite des Beines (26) ausrichtbare Abstandssensoren (12,14)
- und eine mit den Ausgangssignalen der Abstandssensoren (12,14) beaufschlagbare Auswerteeinrichtung (16) zur Bestimmung der Schubladenverschiebung.

2. Meßanordnung nach Anspruch 1, **dadurch gekennzeichnet, daß** ein Abstandssensor (14) gegen die Facies anterior patellae (38) als Bezugsfläche für den Femur (42) und der andere Abstandssensor (12) gegen die Tuberositas tibiae (40) als Bezugsfläche für die Tibia (44) ausrichtbar ist.

3. Meßanordnung nach Anspruch 1 oder 2, **dadurch gekennzeichnet, daß** die Meßstrecken der Abstandssensoren (12, 14) so angeordnet werden können, daß sie parallel zum Tibiaplateau in im wesentlichen antero-posteriorer Richtung verlaufen.

4. Meßanordnung nach einem der Ansprüche 1 bis 3, **dadurch gekennzeichnet, daß** die Auswerteeinrichtung (50,58) die Schubladenverschiebung nach Maßgabe eines Differenzwertes (68) der zeitgleich erfaßten Ausgangssignale (64, 66) der Abstandsensoren (12, 14) bestimmt.

5. Meßanordnung nach einem der Ansprüche 1 bis 4, **dadurch gekennzeichnet, daß** die Abstandssensoren (12,14) vorzugsweise in allen drei Raumrichtungen begrenzt verschiebbar und in ihrer Verschiebelage gestellfest arretierbar an dem Referenzgestell (10) angeordnet sind.

6. Meßanordnung nach einem der Ansprüche 1 bis 5, **dadurch gekennzeichnet, daß** die Abstandssensoren (12,14) einen kapazitiven, induktiven oder als Ohmscher Widerstand ausgebildeten Wegmeßsensor aufweisen, dessen Meßstrecke gegenüber einem gestellfesten Bezugspunkt festgelegt ist.

7. Meßanordnung nach einem der Ansprüche 1 bis 6, **dadurch gekennzeichnet, daß** die Abstandssensoren als Linearpotentiometer (12,14) ausgebildet sind, deren Schleifer (36,38) mit ihren freien Enden federbelastet an die Vorderseite des Beines (26) andrückbar sind.

8. Meßanordnung nach einem der Ansprüche 1 bis 5, **dadurch gekennzeichnet, daß** die Abstandssensoren (12,14) als berührungslos arbeitende Ultraschall-, Infrarot- oder Lasersensoren ausgebildet sind.

9. Meßanordnung nach einem der Ansprüche 1 bis 8, **dadurch gekennzeichnet, daß** das Referenzgestell (10) über ein vorzugsweise als Schaumstoff-Formteil ausgebildetes Stützpolster (22) an der Vorderseite des Unterschenkels (24) abstützbar ist.

10. Meßanordnung nach einem der Ansprüche 1 bis 9, **dadurch gekennzeichnet, daß** das am Unterschenkel (24) tragbare Referenzgestell (10) mittels zirkulär um den Unterschenkel spannbaren, vorzugsweise durch Klettverschlüsse verschließbaren Bändern (28) fixierbar ist.

11. Meßanordnung nach einem der Ansprüche 1 bis 10, **gekennzeichnet durch** eine mit einem Kraftaufnehmer (60) gekoppelte, vorzugsweise eine Bandschlinge aufweisende Kraftübertragungsvorrichtung zur Einleitung einer Kraft in den Unterschenkel (24).

12. Meßanordnung nach einem der Ansprüche 1 bis 11, **dadurch gekennzeichnet, daß** die Auswerteeinrichtung (16) eine tragbare Anzeigeeinheit (50) aufweist, mittels welcher die während eines Meßzyklus erfaßten Werte, insbesondere die Maximalwerte der Ausgangssignale (64,66) der Abstandssensoren (12,14) vorzugsweise auf einem LCD-Display darstellbar sind.

13. Meßanordnung nach einem der Ansprüche 1 bis 12, **gekennzeichnet durch** eine Fernübertragungseinrichtung (52,54) zur Verbindung der Abstandssensoren (12,14) mit der Auswerteeinrichtung (58) über eine drahtlose Sende-/Empfangsstrecke (56).

14. Meßanordnung nach einem der Ansprüche 1 bis 13, **dadurch gekennzeichnet, daß** die Auswerteeinrichtung (50,58) einen über einen Analog/Digital-Wandler mit den digitalisierten Ausgangssignalen der Abstandssensoren (12,14) und gegebenenfalls eines Kraftaufnehmers und/oder Goniometers beaufschlagbaren Mikrocomputer (58) aufweist.

15. Meßanordnung nach einem der Ansprüche 1 bis 14, **gekennzeichnet durch** Oberflächenelektroden zur Ableitung von elektromyographischen Signalen (70,72) insbesondere des M. biceps femoris und M. gastrocnemius.

16. Meßanordnung nach einem der vorhergehenden Ansprüche, wobei mindestens drei Abstandssensoren (12, 14,15) an dem Referenzgestell (10) angeordnet sind, von denen ein erster Abstandssensor (14) im distalen Femurbereich und ein zweiter Abstandssensor (12) im proximalen Tibiabereich gegen die Vorderseite des Beines (26) ausrichtbar ist, **dadurch gekennzeichnet, daß** ein dritter oder weiterer Abstandssensor (15) im horizontalen oder vertikalen Abstand von dem zweiten Abstandssensor (12) gegen den Unterschenkel (24) ausgerichtet ist.

## Claims

1. Measuring device for determining drawer displacement of the tibia (44) in relation to the femur (42) on the leg (26) of a person to be examined comprising a reference framework (10) fixable on the calf (24) of the leg (26), **characterized by**
- two distance sensors (12, 14) which are fixedly mounted, spaced apart from each other, on the reference framework (10) in the proximal area of the tibia and the distal area of the femur, and which can be directed towards the front surface of the leg (26),
- and a drawer displacement analysis device (16) able to receive output signals from the distance sensors (12, 14).

2. Measuring device according to claim 1, **characterized** therein, that one distance sensor (14) is directable against the Facies anterior patellae (38) as reference surface for the femur (42) and the other distance sensor (12) is directable against the Tuberositas tibiae (40) as reference surface for the tibia (44).

3. Measuring device according to claim 1 or 2, **characterized** therein, that the measurement paths of the distance sensors (12, 14) can be arranged in such manner that they run parallel to the tibia plateau in an essentially anterior-posterior direction.

4. Measuring device according to one of claims 1 through 3, **characterized** therein, that a processing unit (50, 58) determines the drawer displacement according to a differential curve (68) of the simultaneously registered output signals (64, 66) of the distance sensors (12, 14).

5. Measuring device according to one of claims 1 through 4, **characterized** therein, that the distance sensors (12, 14) are preferably limitedly movable in all three spatial directions and in their displaced position are fixable on the reference framework (10).

6. Measuring device according to one of claims 1 through 5, **characterized** therein, that the distance sensors (12, 14) are capacitative, inductive or ohmic resistance based odometer sensors, of which the measurement length is predetermined with respect to a frame-fixed reference point.

7. Measuring device according to one of claims 1 through 6, **characterized** therein, that the distance sensors are formed as linear potentiometers (12, 14), of which the sliders (36, 38) are spring biased towards their two free ends and can be urged against the front side of the leg (26).

8. Measuring device according to one of claims 1 through 5, **characterized** therein, that the distance sensors (12, 14) are formed as ultrasonic, infrared or laser sensors for contactless measurement.

9. Measuring device according to one of claims 1 through 8, **characterized** therein, that the reference framework (10) is supported on the front side of the calf (24) by a support cushion (22), preferably of a foam material shaped part.

10. Measuring device according to one of claims 1 through 9, **characterized** therein, that the reference framework (10), which can be worn on the calf (24), is securable by circular closure straps (28) tightenable around the calf, preferably of VELCRO.

11. Measuring device according to one of claims 1 through 10, **characterized by** a force transmission device coupled to a force recorder (60), preferably including a band sling, for introduction of force into the calf (24).

12. Measuring device according to one of claims 1 through 11, **characterized** therein, that the analysis device (16) comprises a hand-held processing unit (50), by means of which the sensed values, particularly the maximal values of the starting signals (64, 66) of the distance sensors (12, 14) sensed during a measurement cycle, can be represented in an LCD-display.

13. Measuring device according to one of claims 1 through 12, **characterized by** a transmitter or receiver module (52, 54) in order to connect the distance sensors (12, 14) with the processing unit (58) over an wireless radio path (56).

14. Measuring device according to one of claims 1 through 13, **characterized** therein, that the processing unit (50, 58) comprises a microcomputer (58), which can be acted upon by the output signals, which may be digitized via an analog/digital converter, of the distance sensors (12, 14) and, in certain cases, the output signals of a force recorder or goniometer.

15. Measuring device according to one of claims 1 through 14, **characterized by** surface electrodes for detection of electromyographic signals (70, 72), in particular of the M. biceps femoris und M. gastrocnemius.

16. Measuring device according to one of the preceeding claims, wherein at least three distance sensors (12, 14, 15) are provided on the reference framework (10), of which a first distance sensor (14) is directable against the front side of the leg (26) in the distal femur area and a second distance sensor (12) in the proximal tibia area, **characterized** therein, that a third or further distance sensor (15) is positioned in horizontal or vertical separation from the second distance sensor (12) and directed against the calf (24).

## Revendications

1. Dispositif de mesure pour la détermination du tiroir ou des signes du tiroir du tibia (44) par rapport au fémur (42) au niveau du membre inférieur (26) d'un sujet d'expérience, avec un bâti de référence (10) pouvant être fixé sur la jambe (24) du membre inférieur (26), **caractérisé par**
- deux capteurs de distance (12, 14) disposés à demeure sur le bâti de référence (10) à distance l'un de l'autre, orientables vers la face antérieure du membre inférieur (26) dans la zone proximale du tibia et la zone distale du fémur
- et un dispositif d'évaluation (16) pouvant recevoir les signaux de sortie des capteurs de distance (12, 14) pour la détermination du tiroir ou des signes du tiroir.

2. Dispositif de mesure selon la revendication 1, **caractérisé par le fait qu'**un des capteurs de distance (14) est orientable vers la face antérieure de la rotule (38) prise comme surface de référence pour le fémur (42) et l'autre capteur de distance (12) est orientable vers la tubérosité tibiale (40) prise comme surface de référence pour le tibia (44).

3. Dispositif de mesure selon la revendication 1 ou 2, **caractérisé par le fait que** les distances de mesure des capteurs de distance (12, 14) peuvent être disposées de manière à être parallèles au plateau tibial et s'étendre essentiellement en direction antéropostérieure.

4. Dispositif de mesure selon l'une des revendications 1 à 3, **caractérisé par le fait que** le dispositif d'évaluation (50, 58) détermine le tiroir ou les signes du tiroir en fonction d'une valeur de différence (68) des signaux de sortie (64, 66) des capteurs de distance (12, 14) mesurés en des temps égaux.

5. Dispositif de mesure selon l'une des revendications 1 à 4, **caractérisé par le fait que** les capteurs de distance (12, 14) sont de préférence disposés sur le bâti de référence (10) de manière à pouvoir être déplacés de façon limitée dans les trois directions de l'espace et bloqués à demeure sur le bâti dans leur position déplacée.

6. Dispositif de mesure selon l'une des revendications 1 à 5, **caractérisé par le fait que** les capteurs de distance (12, 14) présentent un capteur de déplacement capacitif, inductif ou formé d'une résistance ohmique dont la distance de mesure est définie par rapport à un point de référence fixe du bâti.

7. Dispositif de mesure selon l'une des revendications 1 à 6, **caractérisé par le fait que** les capteurs de distance sont formés de potentiomètres linéaires (12, 14) dont les curseurs (36, 38) peuvent être appuyés par leurs extrémités libres, sous l'action de ressorts, sur la face antérieure du membre inférieur (26).

8. Dispositif de mesure selon l'une des revendications 1 à 5, **caractérisé par le fait que** les capteurs de distance (12, 14) sont formés de capteurs sans contact à ultrasons, à infrarouges ou à laser.

9. Dispositif de mesure selon l'une des revendications 1 à 8, **caractérisé par le fait que** le bâti de référence (10) peut être soutenu sur la face antérieure de la jambe (24) par un coussin de soutien (22) formé de préférence d'une pièce moulée en mousse.

10. Dispositif de mesure selon l'une des revendications 1 à 9, **caractérisé par le fait que** le bâti de référence (10) portable sur la jambe (24) peut être fixé au moyen de bandes (28) pouvant être tendues circulairement autour de la jambe, fermant de préférence par des bandes velcro.

11. Dispositif de mesure selon l'une des revendications 1 à 10, **caractérisé par** un dispositif de transmission de force couplé à un capteur de force (60), présentant de préférence une sangle, servant à induire une force dans la jambe (24).

12. Dispositif de mesure selon l'une des revendications 1 à 11, **caractérisé par le fait que** le dispositif d'évaluation (16) présente une unité d'affichage portable (50) au moyen de laquelle les valeurs mesurées pendant un cycle de mesure, en particulier les valeurs maximales des signaux de sortie (64, 66) des capteurs de distance (12, 14), sont représentées de préférence sur un afficheur à cristaux liquides.

13. Dispositif de mesure selon l'une des revendications 1 à 12, **caractérisé par** un dispositif de transmission à distance (52, 54) pour la liaison des capteurs de distance (12, 14) avec le dispositif d'évaluation (58) par une voie d'émission/réception sans fil (56).

14. Dispositif de mesure selon l'une des revendications 1 à 13, **caractérisé par le fait que** le dispositif d'évaluation (50, 58) présente un microordinateur (58) pouvant recevoir par l'intermédiaire d'un convertisseur analogique-numérique les signaux de sortie numérisés des capteurs de distance (12, 14) et le cas échéant d'un capteur de force et/ou d'un goniomètre.

15. Dispositif de mesure selon l'une des revendications 1 à 14, **caractérisé par** des électrodes de surface pour la mesure des signaux électromyographiques (70, 72) en particulier du muscle du biceps fémoral et du muscle gastrocnémien.

16. Dispositif de mesure selon l'une des revendications précédentes, dans lequel sont disposés sur le bâti de référence (10) au moins trois capteurs de distance (12, 14, 15) dont un premier capteur de distance (14) est orientable vers la face antérieure du membre inférieur (26) dans la zone distale du fémur, un deuxième capteur de distance (12) est orientable vers ladite face (26) dans la zone proximale du tibia, **caractérisé par le fait qu'**un troisième ou autre capteur de distance (15) est orienté vers la jambe (24) à une distance horizontale ou verticale du deuxième capteur de distance (12).
